(19) Europäisches Patentamt / European Patent Office / Office européen des brevets

(11) **EP 2 297 717 B1**

(12) **EUROPÄISCHE PATENTSCHRIFT**

(45) Veröffentlichungstag und Bekanntmachung des Hinweises auf die Patenterteilung:
**31.10.2012 Patentblatt 2012/44**

(21) Anmeldenummer: 08761015.0

(22) Anmeldetag: **13.06.2008**

(51) Int Cl.:
*G08B 21/14* *(2006.01)*

(86) Internationale Anmeldenummer:
**PCT/EP2008/057494**

(87) Internationale Veröffentlichungsnummer:
**WO 2009/149767 (17.12.2009 Gazette 2009/51)**

(54) **BESTIMMUNG EINER ALARMIERUNGSZEIT EINES GEFAHRMELDERS**

DETERMINATION OF AN ALARM-ISSUING TIME OF AN ALARM DEVICE

DÉTERMINATION DU DÉLAI DE RÉPONSE D'UN AVERTISSEUR DE DANGER

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(43) Veröffentlichungstag der Anmeldung:
**23.03.2011 Patentblatt 2011/12**

(73) Patentinhaber: **Siemens Aktiengesellschaft
80333 München (DE)**

(72) Erfinder:
• **FORSTER, Martin
CH-8645 Jona (CH)**
• **DURIC, Aleksandar
CH-6300 Zug (CH)**

(56) Entgegenhaltungen:
**US-A- 5 276 434    US-A- 5 786 768**

## Beschreibung

Die vorliegende Erfindung betrifft das technische Gebiet der Gefahrmeldetechnik. Die vorliegende Erfindung betrifft insbesondere ein Verfahren zum Bestimmen eines Auslösezeitpunkts für einen Gefahrenmelder, dessen Auslösezeitpunkt von einem erfassten Messwert, welcher für ein Gefahrenpotential innerhalb eines Überwachungsbereiches indikativ ist, abhängig ist. Die vorliegende Erfindung betrifft ferner einen Gefahrenmelder mit einer Detektionseinrichtung zum Erfassen eines derartigen Messwertes und mit einer Auswerteinrichtung, welche zur Durchführung des genannten Verfahrens eingerichtet ist. Ferner betrifft die vorliegende Erfindung ein Programm-Element zum Bestimmen eines Auslösezeitpunkts für eine Alarmmeldung eines Gefahrenmelders, welches in eine Auswerteeinheit des Gefahrenmelders geladen werden kann und eine Durchführung des oben genannten Verfahrens veranlassen kann.

Um ein unerwünschtes Auftreten einer Gefahrensituation wie beispielsweise ein Austreten und/oder ein Entstehen eines gefährlichen Gases frühzeitig zu erkennen, werden häufig Gefahrmelder verwendet, die in einem gefahrenüberwachten Bereich beispielsweise innerhalb eines Gebäudes an geeigneten Stellen angebracht sind. Abhängig von der jeweils zu erfassenden Gefahrensituation ist ein Gefahrmelder mit einem geeigneten Detektor ausgestattet, welcher basierend auf einer physikalischen Messung möglichst frühzeitig die Gefahrensituation erkennen kann. Im Falle einer durch Gas verursachten Gefahrensituation ist der Detektor typischerweise ein Gassensor, welcher sensitiv für ein zu detektierendes Gas oder für mehrere zu detektierende Gase ist.

Ein Gefahrmelder kann auch ein Teil eines Gefahrmeldesystems oder eines umfassenden Gebäudemanagementsystems sein, welches neben einer Zentrale mehrere als Peripheriegeräte ausgebildete Gefahrmelder aufweist. Die Peripheriegeräte können mit der Zentrale über eine leitungsgebundene oder eine drahtlose Kommunikationsverbindung direkt oder eine indirekt verbunden sein.

Aus der US 4,088,986 ist ein Gefahrmeldesystem mit einer Zentrale und einer Mehrzahl von Gassensoren bekannt. Wenn verschiedene Gassensoren jeweils eine gefährliche, oberhalb von vorgegebenen Schwellenwerten liegende Gaskonzentration an die Zentrale melden, wird ein Alarm ausgegeben.

Aus der WO 2005/119618 A2 ist ein Algorithmus zum Ausgeben eines Feueralarms bekannt, welcher als Eingangsgrößen die Ausgangssignale eines Rauch-Detektors, eines CO-Gassensors und eines $CO_2$-Gassensors verwendet. Wenn die Anstiege der verschiedenen Sensoren einzeln oder im Verbund bestimmte Schwellenwerte überschreiten, dann wird eine Alarmmeldung initiiert.

Aus der EP 0 880 764 B1 ist ein Multi-Signatur Brandmelder bekannt, bei dem ein für eine Rauchkonzentration indikatives Signal und ein für eine ggf. gefährliche Gaskonzentration indikatives Signal miteinander multipliziert werden. Wenn bei dieser Multiplikation ein bestimmter Wert überschritten wird, so wird ein Alarm ausgegeben. Ferner wird ein Alarm ausgegben, wenn lediglich eine zeitliche Änderung der Gaskonzentration einen bestimmten Wert überschreitet.

Gasmelder oder Gas-Rauchmelder unterliegen jedoch gesetzlichen Vorschriften. Diese Vorschriften, die üblicherweise in einer Norm festgehalten sind, muss ein entsprechender Melder erfüllen, bevor er in dem jeweiligen Land eine Zulassung erhält.

In Europa gibt es beispielsweise für die Detektion von Kohlenmonoxid (CO) in Wohnhäusern vorgegebene Prüfverfahren und Anforderungen an das Betriebsverhalten von CO Gasmeldern. Diese sind in der Norm mit der Nummer EN 50291 beschrieben.

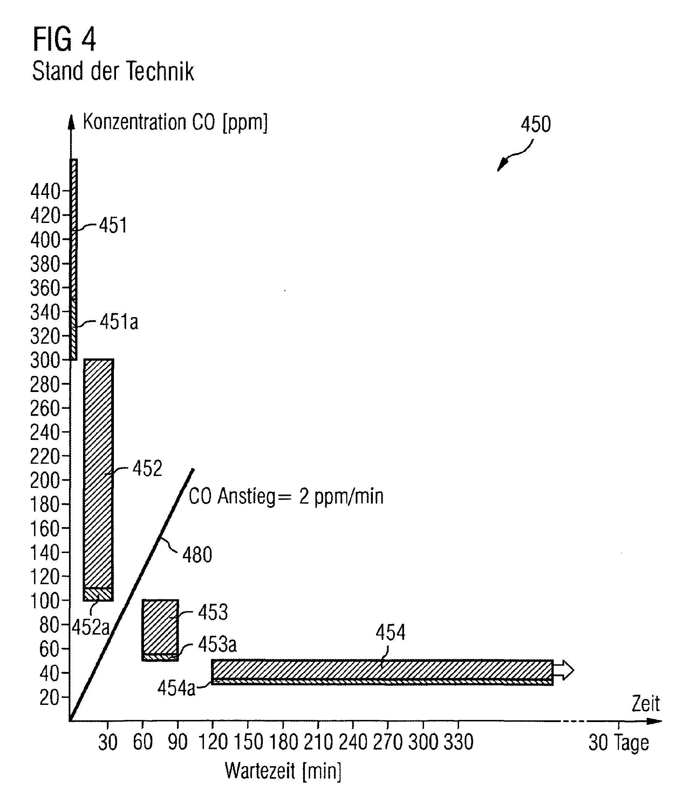

Figur 4 zeigt in einem Diagramm 450 einige Zulassungsbedingungen der Norm EN 50291 für einen CO-Gasmelder. Die Norm EN 50291 definiert einige Alarmierungsbedingungen, eine erste Alarmierungsbedingung 451a, eine zweite Alarmierungsbedingung 452a, eine dritte Alarmierungsbedingung 453a und eine vierte Alarmierungsbedingung 454a. Diese Alarmierungsbedingungen geben jeweils für einen Messwert für die CO Konzentration eine bestimmte minimale Wartezeit an, die nach einem Auftreten des Messwertes abgewartet werden muss, bevor eine Alarmmeldung erfolgen darf. Ebenso geben diese Alarmierungsbedingungen auch eine maximale Wartezeit an, innerhalb der nach einem Auftreten eines entsprechenden Messwertes spätestens eine Alarmmeldung erfolgen muss. Die Wartezeiten sind in dem Diagramm 450 auf der Abszisse aufgetragen. Die Messwerte für die CO Konzentration sind auf der Ordinate aufgetragen.

Die erste Alarmierungsbedingung 451a schreibt vor, dass bei Auftreten einer CO-Konzentration von $330\pm30$ ppm spätestens nach 3 Minuten eine Alarmmeldung erfolgen muss. Die zweite Alarmierungsbedingung 452a schreibt vor, dass bei Auftreten einer CO-Konzentration von $110\pm10$ ppm (a) frühestens nach 10 Minuten eine Alarmmeldung erfolgen darf und (b) spätestens bis 40 Minuten nach dem Auftreten der entsprechenden CO-Konzentration eine Alarmmeldung erfolgen muss. Die dritte Alarmierungsbedingung 453a schreibt vor, dass bei Auftreten einer CO-Konzentration von $55\pm5$ ppm (a) frühestens nach einer Wartezeit von 60 Minuten eine Alarmmeldung erfolgen darf und (b) spätestens bis 90 Minuten nach dem Auftreten der entsprechenden CO-Konzentration eine Alarmmeldung erfolgen muss. Die vierte Alarmierungsbedingung 454a schreibt vor, dass bei Auftreten einer CO-Konzentration von $33\pm3$ ppm frühestens nach einer Wartezeit von 120 Minuten eine Alarmmeldung erfolgen darf. In der Praxis werden für jede der Alarmierungsbedingungen 454a, 453a und 452a der entsprechende Messwertbereich hin bis zu dem unteren Messwert der in Bezug auf den Messwert nächst höheren Alarmierungsbedingung 453a, 452a und 451a erweitert. Dabei ergeben sich dann die in dem Diagramm 450 dargestellten möglichen Alarmierungsbereiche 451, 452, 453 und 454.

Es wird darauf hingewiesen, dass es für andere Länder auch andere Normen gibt, die für die verschiedenen CO-Konzentrationen andere Wartezeiten bis zum Auslösen einer Alarmmeldung vorschreiben. In diesem Zusammenhang ist insbesondere die in den USA gültige Norm UL 2034 zu erwähnen.

Um einen Gasmelder zu schaffen, welcher die jeweils gültige Norm erfüllt, muss in einer Auswerteinheit des Gasmelders ein entsprechender Algorithmus implementiert werden, welcher jedem beliebigen Messwert eine bestimmte mit der Norm kompatible Wartezeit für das Auslösen einer Alarmmeldung vorschreibt.

Für zeitlich weitgehend konstante Gaskonzentrationen können verschiedene länderspezifische Normen durch entsprechend länderspezifisch angepasste Algorithmen noch relativ einfach erfüllt werden. Sobald aber ansteigende oder abfallende Gaskonzentrationen auftreten, ergeben sich Situationen, die im Hinblick auf eine eindeutige Zuordnung einer Wartezeit zu einem bestimmten Messwert problematisch sein können. Diese Problematik wird im Folgenden anhand eines beispielhaften zeitlichen Anstiegs der CO-Konzentration von 2 ppm pro Minute verdeutlicht. Dieser Anstieg ist in dem Diagramm 450 mittels eine geraden Anstiegskurve 480 dargestellt.

Zur Zeit $t = 15$ Minuten hat die CO-Konzentration einen Wert von 30 ppm erreicht. Ein Alarm müsste dann zur Zeit $t\_alarm = 15 + 120 = 135$ Minuten erfolgen. Zur Zeit $t = 24$ Minuten hat die CO-Konzentration einen Wert von 48 ppm erreicht und der Alarm müsste immer noch erst zur Zeit $t\_alarm = 15 + 120 = 135$ Minuten erfolgen. Eine Minute später, zur Zeit $t = 25$ Minuten, hat die CO-Konzentration einen Wert von 50 ppm erreicht und ab jetzt müsste der Alarm plötzlich schon zur Zeit $t\_alarm = 25 + 60 = 85$ Minuten erfolgen. Wie aus Figur 4 ersichtlich, gibt es analoge Sprünge für den geforderten Alarmierzeitpunkt beim Übergang von $t = 49$ Minuten (Alarm muss nach 85 Minuten erfolgen) auf $t = 50$ Minuten (Alarm muss nach $50 + 10 = 60$ Minuten erfolgen).

Der Erfindung liegt die Aufgabe zugrunde, die Berechnung von Alarmzeiten eines Gefahrenmelders dahingehend zu verbessern, dass bei veränderlichen Messwerten Sprünge bei der Bestimmung des Alarmierungszeitpunkts vermieden werden können und dass die Alarmierung so früh wie möglich, aber immer noch innerhalb der von der Norm geforderten Zeitspanne erfolgt.

Diese Aufgabe wird gelöst durch die Gegenstände der unabhängigen Patentansprüche. Vorteilhafte Ausführungsformen der vorliegenden Erfindung sind in den abhängigen Ansprüchen beschrieben.

Gemäß einem ersten Aspekt der Erfindung wird ein Verfahren zum Bestimmen eines Auslösezeitpunkts für eine Alarmmeldung eines Gefahrenmelders beschrieben. Das Verfahren weist auf (a) ein Erfassen eines Messwertes zu einem Messzeitpunkt, wobei der Messwert für ein Gefahrenpotential innerhalb eines Überwachungsbereiches indikativ ist, (b) ein Ermitteln einer Wartezeit mittels einer Funktion, welche für eine Vielzahl von unterschiedlichen Messwerten jeweils eine zugeordnete Wartezeit angibt und welche einen stetigen Verlauf aufweist, und (c) ein Bestimmen des Auslösezeitpunkts basierend auf dem Messzeitpunkt und der ermittelten Wartezeit.

Unter einer stetigen Funktion werden im Rahmen dieser Anmeldung auch Funktionen verstanden, deren Verlauf stetig ist und deren erste Ableitung entweder ebenfalls einen stetigen Verlauf oder aber einen unstetigen Verlauf aufweist. Dies bedeutet, dass auch Funktionen mit Knickpunkten, die eine sich plötzlich ändernde erste Ableitung bewirken, ebenfalls stetige Funktionen im Sinne dieser Anmeldung darstellen.

Dem beschriebenen Verfahren liegt die Erkenntnis zugrunde, dass mittels einer stetigen Funktion zur Ermittlung für jeden beliebigen erfassten Messwert eine geeignete Wartezeit bestimmt werden kann, wobei die Abhängigkeit der Wartezeit von dem Messwert keine Sprünge bzw. Unstetigkeiten aufweist. Dies kann bedeuten, dass bei einer fiktiven geringfügigen Erhöhung des Messwertes auch die entsprechende Wartezeit sich nur geringfügig ändert.

Die Eliminierung von Unstetigkeiten bei der messwertabhängigen Ermittlung einer geeigneten Wartezeit hat den Vorteil, dass es insbesondere bei zeitlich veränderlichen Messwerten bei der Ermittlung der Wartezeit und/oder bei der Bestimmung des Auslösezeitpunkts zu keinen zweideutigen Ergebnissen kommt. Damit kann eine besonders zuverlässige Bestimmung der Auslösezeitpunkte von Alarmmeldungen gewährleistet werden.

Die beschriebene stetige Funktion kann in einem zweidimensionalen Koordinatensystem, in dem auf der einen Achse die unterschiedlichen Messwerte und auf der anderen Achse die jeweils zugeordneten Wartezeiten aufgetragen sind, visualisiert werden. Die stetige Funktion kann dabei derart gewählt sein, dass automatisch eine Mehrzahl von Normen, die für eine Zulassung eines entsprechenden Gefahrenmelders erfüllt werden müssen, auch tatsächlich erfüllt wird. Unter dem Begriff Norm ist in diesem Zusammenhang eine gesetzliche Vorschrift zu verstehen, welche typischerweise für unterschiedliche Länder unterschiedlich ist und welche Kriterien festlegt, ob und wann bei einem bestimmten Messwert von einem Gefahrenmelder, welcher nach dem beschriebenen Verfahren arbeitet, ein Alarm ausgelöst werden muss.

Durch die Verwendung der beschriebenen stetigen Funktion zum Ermitteln einer Wartezeit kann somit für verschiedenartige Gefahrenmelder eine einfache und insbesondere eine allgemeingültige Methode bereitgestellt werden, mit der sowohl für zeitlich schwach als auch für zeitlich stark veränderliche Messwerte eine zuverlässige Bestimmung des Auslösezeitpunkts für eine Alarmmeldung gewährleistet werden kann. Ferner können durch eine geeignete Wahl der stetigen Funktion und durch eine entsprechende Implementierung des beschriebenen Verfahrens in einem Gefahrenmelder alle möglichen Normen für den Gefahrenmelder erfüllt werden.

Die stetige Funktion kann beispielsweise durch eine mathematische Anpassung erfolgen, bei der ein oder mehrere Parameter der Funktion auf geeignete Weise angepasst werden, so dass auf alle Fälle die von den relevanten Normen geforderten Sensitivitäten des entsprechenden Gefahrenmelders erfüllt werden.

Gemäß einem Ausführungsbeispiel der Erfindung ist die Funktion derart beschaffen, dass sie innerhalb eines durch eine Norm vorgegebenen Wartezeitbereiches einen ebenfalls durch die Norm vorgegebenen zugehörigen unteren Grenzwert für den Messwert überschreitet.

In diesem Sinn kann durch die Norm beispielsweise ein Alarmierungszeitbereich vorgegeben sein, welcher in dem oben beschriebenen zweidimensionalen Koordinatensystem die Form eines Rechtecks aufweist. Sofern in diesem Koordinatensystem auf der Ordinate die Messwerte und auf der Abszisse die entsprechenden Wartezeiten aufgetragen sind, wird der untere Grenzwert für den Messwert durch die untere horizontale Begrenzungslinie des Alarmierungszeitbereiches bestimmt. Diese horizontale Begrenzungslinie wird dann innerhalb des vorgegebenen Wartezeitbereiches von der Funktion geschnitten.

Der beschriebene untere Grenzwert für den Messwert, welcher innerhalb des vorgegebenen Wartezeitbereichs von der stetigen Funktion zumindest einmal angenommen werden muss, stellt somit für eine Bestimmung der stetigen Funktion eine zu erfüllende Bedingung dar. Diese Bedingung kann beispielsweise durch eine geeignete Anpassung von einem oder von mehreren Parametern, welche die stetige Funktion charakterisieren, erfüllt werden.

Gemäß einem weiteren Ausführungsbeispiel der Erfindung ist die Funktion derart beschaffen, dass sie einen wartezeitunabhängigen minimalen Grenzwert für den Messwert nicht unterschreitet.

Ein wartezeitunabhängiger minimaler Grenzwert stellt eine absolute untere Schranke für den Messwert dar, unterhalb der eine Alarmauslösung nicht erfolgen darf. Ein derartiger minimaler Grenzwert kann ebenfalls von gesetzlichen Normen vorgeschrieben sein, um unabhängig von einem zeitlichen Verlauf des Messwertes, welcher jedoch stets kleiner ist als der minimale Grenzwert, ungewollte Falschalarme zu vermeiden. Damit wird der allgemein bekannten Tatsache Rechnung getragen, dass die relativen statistischen und/oder durch eine Detektionseinrichtung verursachten Unsicherheiten bei der Erfassung eines Messwertes dann am größten sind, wenn die erfassten Messwerte sehr klein sind.

In dem oben beschriebenen Koordinatensystem, bei dem auf der Abszisse die Wartezeit und auf der Ordinate die Messwerte aufgetragen sind, stellt der wartezeitunabhängige minimale Grenzwert für den Messwert eine horizontale untere Begrenzungslinie dar. Dieser darf sich die stetige Funktion nur asymptotisch nähern und darf sie nicht schneiden.

An dieser Stelle wird darauf hingewiesen, dass die stetige Funktion selbstverständlich auch in einem Koordinatensystem visualisiert werden kann, in dem auf der Ordinate die Wartezeit und auf der Abszisse die Messwerte aufgetragen sind. An dem oben beschriebenen technischen Sachverhalt und an den oben beschriebenen Vorteilen ändert sich durch die geänderte Visualisierung jedoch nichts.

Gemäß einem weiteren Ausführungsbeispiel der Erfindung ist die Funktion derart beschaffen, dass sie innerhalb eines durch eine weitere Norm vorgegebenen Messwertbereiches einen ebenfalls durch die weitere Norm vorgegebenen zugehörigen frühen Grenzwert für die Wartezeit überschreitet. Auch in diesem Fall kann durch die weitere Norm ein Alarmierungszeitbereich vorgegeben sein, welcher in dem oben beschriebenen zweidimensionalen Koordinatensystem zum Beispiel die Form eines Rechtecks aufweist. Sofern auch in diesem Fall auf der Ordinate der Messwert und auf der Abszisse die Wartezeit aufgetragen ist, wird der frühe Grenzwert für die Wartezeit durch die linke zum Beispiel vertikale Begrenzungslinie des entsprechenden Alarmierungszeitbereiches bestimmt. Diese zum Beispiel vertikale Begrenzungslinie wird dann von der stetigen Funktion geschnitten.

Gemäß einem weiteren Ausführungsbeispiel der Erfindung ist die Funktion derart beschaffen, dass sie sich innerhalb eines Messwertbereiches mit besonders hohen Messwerten asymptotisch an die Wartezeit Null annähert. Auf diese Weis kann sichergestellt werden, dass ab einer bestimmten Höhe eines erfassten Messwertes unabhängig von dem vorherigen zeitlichen Verlauf des Messwertes, welcher in diesem Fall typischerweise einen sehr schnellen Anstieg aufweist, sofort eine Alarmmeldung ausgelöst wird. Auch dieses Verhalten kann von gesetzlichen Normen verlangt werden, um im Falle eines abrupten Messwertanstiegs eine unverzügliche Alarmauslösung zu gewährleisten.

In dem oben beschriebenen Koordinatensystem, bei dem auf der Ordinate die Messwerte und auf der Abszisse die zugehörigen Wartezeiten aufgetragen sind, stellt eine entsprechende Norm, die verlangt, dass bei hohen Messwerten sofort ein Alarm erfolgen muss, einen Alarmierungszeitbereich dar, dessen vertikale linke Begrenzungslinie von der Schwellenfunktion nicht geschnitten, sondern nur berührt werden darf.

Gemäß einem weiteren Ausführungsbeispiel der Erfindung wird die Funktion durch einen oder durch mehrere Parameter und durch eine Funktionsvariable bestimmt. Die Art der Funktion kann dabei frei gewählt werden, so dass die Funktion die die durch jede relevante Norm vorgegebenen Alarmierungszeitbereiche mit jeweils angepassten Parametern im obigen Sinne approximiert bzw. annähert. Die Parameter können auch als Koeffizienten bezeichnet werden. Dies kann bedeuten, dass für jede beliebige Norm beispielsweise im Rahmen einer Anpassungsprozedur optimale Parameter bzw. Koeffizienten bestimmt werden.

Hinsichtlich der Anzahl der für die Beschreibung der Funktion erforderlichen Parameter gibt es keine prinzipielle Obergrenze. Je höher die Anzahl der verwendeten Parameter ist, desto genauer kann die Funktion an verschiedene gesetzliche Normen und/oder gewünschte Alarmierungszeitbereiche angepasst werden. Selbstverständlich steigt mit der Anzahl der verwendeten Parameter auch die Komplexität insbesondere einer geeigneten Anpassungsprozedur zur Festlegung der Parameter für einen konkreten Einsatzfall. In der Praxis hat es sich als einen guten Kompromiss zwischen der Komplexität und der erreichbaren Genauigkeit der stetigen Funktion herausgestellt, wenn drei, vier oder fünf Parameter zur Beschreibung der Funktion verwendet werden.

Selbstverständlich hängt die Anzahl an bevorzugt zu verwendenden Parametern auch von der Art der Funktion ab. Die Funktion kann eine oder mehrere Bestandteile haben. Diese Bestandteile können beispielsweise ein Polynom, eine Hyperbel, eine trigonometrische Funktion, eine Logarithmusfunktion, eine Exponentialfunktion etc. aufweisen. Selbstverständlich kann die Funktion auch eine Kombination aus verschiedenen der genannten oder weiteren, in dieser Anmeldung nicht erwähnten mathematischen Bestandteile aufweisen.

Gemäß einem weiteren Ausführungsbeispiel der Erfindung sind der oder die Parameter derart gewählt, dass die Funktion einen von der Wartezeit unabhängigen konstanten Messwert ergibt, welcher einen Grenzmesswert darstellt. Dieser Grenzmesswert kann insbesondere durch einen Parameter beschrieben werden, welcher innerhalb der Funktion eine Konstante darstellt. Dies bedeutet, dass bei einem Erreichen dieses Grenzmesswertes sofort ein Alarm ausgelöst wird. Dies kann daran gesehen werden, dass in dem oben beschriebenen zweidimensionalen Koordinatensystem einem Messwert, welcher mit dem Grenzmesswert identisch ist, unter anderem auch die Wartezeit Null zugeordnet ist.

Gemäß einem weiteren Ausführungsbeispiel der Erfindung ist die Funktionsvariable die Wartezeit oder der Messwert. Dies bedeutet, dass die stetige Funktion in dem oben beschriebenen zweidimensionalen Koordinatensystem aufgetragen werden kann, wobei es unerheblich ist, ob die Wartezeit auf der Abszisse oder der Ordinate und der Messwert auf der Ordinate oder der Abszisse aufgetragen ist.

Falls die Wartezeit auf der Abszisse und der Messwert auf der Ordinate aufgetragen ist, dann kann die einem erfassten Messwert zugeordnete Wartezeit auf einfache Weise dadurch bestimmt werden, dass die oben genannte stetige Funktion invertiert und der erfasste Messwert in die invertierte Funktion eingesetzt wird. Der Auslösezeitpunkt kann sich dann durch eine einfache Addition des entsprechenden Messzeitpunktes und der entsprechend bestimmten Wartezeit ergeben. Der Auslösezeitpunkt kann mathematisch durch folgende Gleichung dargestellt werden:

$$\text{t\_alarm} = \text{t\_mess} + f^{-1}(a, b, c, ..., \text{Messwert})$$

Dabei steht

t_alarm für den Auslösezeitpunkt;
t_mess für den Messzeitpunkt;
$f^{-1}$ für die invertierte stetige Funktion f;
a, b, c, ... für einzelne Parameter der Funktion f bzw. $f^{-1}$; und
Messwert für den zur Zeit t_mess erfassten Messwert.

Falls der Messwert auf der Abszisse und die Wartezeit auf der Ordinate aufgetragen ist, dann kann die einem erfassten Messwert zugeordnete Wartezeit einfach dadurch bestimmt werden, dass der erfasste Messwert in eine stetige Funktion g eingesetzt wird. Der Auslösezeitpunkt kann sich dann in entsprechender Weise durch eine einfache Addition des entsprechenden Messzeitpunktes und der entsprechend bestimmten Wartezeit ergeben. Die Umkehrfunktion muss hier also nicht benutzt werden. Dies kann mathematisch durch folgende Gleichung dargestellt werden:

$$\text{t\_alarm} = \text{t\_mess} + g\,(a, b, c, ..., \text{Messwert})$$

Dabei steht

t_alarm für den Auslösezeitpunkt;
t_mess für den Messzeitpunkt;
g für die stetige Funktion;
a, b, c, ... für einzelne Parameter der Funktion g; und Messwert für den zur Zeit t_mess erfassten Messwert.

Gemäß einem weiteren Ausführungsbeispiel der Erfindung weist die Funktion mehrere Teilabschnitte auf, wobei jeweils zwei benachbarte Teilabschnitte an einer Knickstelle der Funktion miteinander verbunden sind.

Die linearen Teilfunktionen können dabei durch bestimmte Punkte von normierten Alarmierungsbedingungen definiert sein, wobei jeder Punkt durch einen Messwert und eine zugeordnete Wartezeit definiert ist. Die Verwendung einer Funktion mit mehreren Teilabschnitten, welche auch als segmentierte Funktion bezeichnet werden kann, hat den Vorteil, dass beliebige normierte Alarmierungsbedingungen besonders gut approximiert werden können. Dabei kann die Anzahl der für eine gute Approximation erforderlichen Segmente von der Anzahl und von der Verteilung der einzelnen Alarmierungsbedingungen innerhalb eines zweidimensionalen Koordinatensystems abhängen, bei dem auf der einen Achse die unterschiedlichen Messwerte und auf der anderen Achse die jeweils zugeordneten Wartezeiten aufgetragen sind.

Gemäß einem weiteren Ausführungsbeispiel der Erfindung weist das Verfahren ferner auf (a) ein Erfassen eines weiteren Messwertes zu einem weiteren Messzeitpunkt, wobei auch der weitere Messwert für das Gefahrenpotential innerhalb des Überwachungsbereiches indikativ ist, (b) ein Ermitteln einer weiteren Wartezeit mittels der Funktion, und (c) ein Bestimmen eines weiteren Auslösezeitpunkts basierend auf dem weiteren Messzeitpunkt und der ermittelten weiteren Wartezeit.

Eine wiederholte Durchführung des gesamten Verfahrens kann in regelmäßigen zeitlichen Intervallen erfolgen. Abhängig von der Stärke von zu erwartenden Änderungen des Messwertes kann die Messwerterfassung und ggf. auch die nachfolgende Messwertauswertung zum Zwecke der Bestimmung des weiteren Auslösezeitpunkts oder von weiteren Auslösezeitpunkten in unterschiedlichen Zeitabständen erfolgen. Als geeignet hat sich beispielsweise eine Zeitdifferenz von zwei Sekunden erwiesen. Jedoch kann das Verfahren auch in beliebig anderen zeitlichen Abständen wiederholt durchgeführt werden.

Die weitere Messwerterfassung und eine nachfolgende weitere Messwertauswertung zum Zwecke der Bestimmung einer weiteren Wartezeit bzw. eines weiteren Auslösezeitpunktes müssen nicht zwangsläufig bedeuten, dass die vorhergehende Bestimmung der Wartezeit bzw. des Auslösezeitpunktes nicht mehr relevant ist. Vielmehr kann das bisherige Verfahren zusammen mit dem weiteren Verfahren weiter ausgeführt werden. Dies bedeutet, dass zwei Auslösezeitpunkte bestimmt werden. Die tatsächliche Alarmauslösung kann dann insbesondere zu dem frühesten Auslösezeitpunkt bestimmt werden.

Für zeitlich variierende Messwerte kann auch ständig ein neuer Auslösezeitpunkt bestimmt werden. Wenn ein Auslösezeitpunkt kleiner ist als oder gleich ist wie der Zeitwert der aktuellen Zeit, dann wird eine Alarmmeldung ausgelöst.

Gemäß einem weiteren Ausführungsbeispiel der Erfindung unterscheidet sich der weitere Messwert zumindest um einen vorgegebenen Wert von dem Messwert. Dies kann bedeuten, dass das weitere Verfahren erst dann durchgeführt wird, wenn (a) der Messwert zeitlich nicht konstant ist und wenn (b) zwischen dem Messzeitpunkt und dem weiteren Messzeitpunkt eine bestimmte Zeitdauer vergangen ist, so dass sich ein geforderter Mindestunterschied zwischen dem Messwert und dem weiteren Messwert einstellt.

Der vorgegebene, einen Messwertunterschied beschreibende Wert kann dabei ein absoluter oder ein relativer Wert sein. Bei einem absoluten Messwertunterschied unterscheidet sich der weitere Messwert von dem Messwert um zumindest einen festen Wert, der von der Höhe des Messwertes unabhängig ist. Bei einem relativen Messwertunterschied unterscheidet sich der weitere Messwert von dem Messwert um einen von der Höhe des Messwertes und/oder des weiteren Messwertes abhängigen Wert.

Gemäß einem weiteren Ausführungsbeispiel der Erfindung wird (a) neben dem weiteren Auslösezeitpunkt auch weiterhin der Auslösezeitpunkt berücksichtigt, falls der weitere Messwert größer ist als der Messwert, und (b) der Auslösezeitpunkt verworfen, falls der weitere Messwert kleiner ist als der Messwert.

Dies kann bedeuteten, dass im Falle eines Messwertanstiegs eine Mehrzahl von Auslösezeitpunkten berücksichtigt wird, wobei die tatsächliche Alarmauslösung dann insbesondere zu dem frühesten Auslösezeitpunkt erfolgt. Damit kann sichergestellt werden, dass unter keinen Umständen eine verspätete Alarmauslösung erfolgt.

Falls jedoch nach einem Messwertanstieg ein Messwertabfall folgt und der Messwert danach auf einem insbesondere für Personen gesundheitlich unbedenklichen Niveau verbleibt, kann durch die Löschung bzw. durch die Verwerfung zumindest des ursprünglichen Auslösezeitpunkts eine nicht erforderliche Alarmmeldung vermieden werden. Dies gilt jedenfalls dann, wenn Wartezeiten oberhalb einer vergleichsweise sehr langen Zeitspanne nicht berücksichtigt werden und somit zu keiner Alarmauslösung führen und/oder wenn den unbedenklichen Messwerten eine unendliche Wartezeit zugeordnet ist.

Gemäß einem weiteren Ausführungsbeispiel der Erfindung ist der Messwert indikativ für die Konzentration eines Gases. Das Gas kann jeder beliebige gasförmige Stoff sein, welcher potentiell eine Gefährdung für Mensch und/oder Maschinen darstellen könnte. Insbesondere kann es sich bei dem Gas um Kohlenmonoxid handeln, welches für den Menschen nicht erkennbar ist und trotzdem ab einer gewissen Konzentration für den Menschen sehr gefährlich sein kann.

Zur Erfassung der Gaskonzentration kann jeder für das betreffende Gas geeignete Gassensor verwendet werden. Dazu zählen beispielsweise elektrochemische Gassensoren, biochemische Gassensoren, Infrarot- Gassensoren, massensensitive Gassensoren und/oder thermochemische Gassensoren. Der Gassensor kann auch als Halbleiterbauelement aufgebaut sein.

Das Gas kann auch ein Stoff sein, dessen Abwesenheit zu einer Gefährdung von Menschen und/oder von Maschinen führen könnte. In diesem Fall wird eine dem Messwert zugeordnete Wartezeit mit abnehmendem Messwert immer kürzer.

An dieser Stelle wird darauf hingewiesen, dass der Messwert und ggf. der weitere Messwert auch für beliebige andere Gefährdungspotentiale indikativ sein kann. So kann der Messwert beispielsweise die Konzentration von Rauch innerhalb eines durch den Gefahrenmelder überwachten Gefahrenbereiches beschreiben. Außerdem kann der Messwert ein Temperaturmesswert sein. Dies macht insbesondere dann Sinn, wenn eine Temperaturänderung beispielsweise durch eine chemische Reaktion verursacht sein kann, mit der für den Menschen giftige Stoffe freigesetzt werden.

Der Messwert kann ferner indikativ sein für die Luftfeuchtigkeit beispielsweise innerhalb einer Lagerstätte für Gemüse und/oder Blumen. Außerdem kann der Messwert ein erfasster Druck sein, welcher in Flüssigkeiten oder Gasen einer Hydraulikanlage vorhanden ist. Auch eine Windstärke beispielsweise im Zusammenhang mit Windturbinen, die Umdrehungszahl eines beliebigen Rotors oder eine Dehnung an einem Gebäude oder einer Brücke können jeweils ein Messwert sein, der für ein bestimmtes Gefährdungspotential indikativ ist.

Unabhängig von der Art des Messwertes gilt, dass je größer das Gefährdungspotential einer gemessenen Größe ist, umso kürzer sollte die Zeitspanne sein, während der das Gefährdungspotential ohne eine Alarmmeldung auftreten darf. Dies bedeutet, dass die Zeitspanne bis hin zu einer Alarmierung umso kleiner sein muss, je größer das jeweilige Gefährdungspotential ist.

Es wird ferner darauf hingewiesen, dass eine Alarmauslösung auch noch mit anderen Messwerten korreliert werden kann. So ist es beispielsweise durchaus denkbar, dass das beschriebene Verfahren in einem sog. Multikriterienmelder beispielsweise im Zusammenhang mit einer erfassten Gaskonzentration angewendet wird, wobei der durch das beschriebene Verfahren bestimmte Auslösezeitpunkt noch durch einen physikalisch anderen Messwert, beispielsweise eine Rauchkonzentration, eine Temperatur, einen Flüssigkeitspegel, etc, modifiziert werden kann.

Gemäß einem weiteren Aspekt der Erfindung wird ein Gefahrenmelder zum Auslösen einer Alarmmeldung beschrieben. Der Gefahrenmelder weist auf (a) eine Detektionseinrichtung, eingerichtet zum Erfassen eines Messwertes, welcher für ein Gefahrenpotential innerhalb eines Überwachungsbereiches indikativ ist, und (b) eine Auswerteeinrichtung, welche mit der Detektionseinrichtung gekoppelt ist und welche derart eingerichtet ist, dass das Verfahren nach einem der vorangehenden Ansprüche ausführbar ist.

Dem beschriebenen Gefahrenmelder liegt die Erkenntnis zugrunde, dass das oben erläuterte Verfahren, welches zum Bestimmen eines geeigneten Auslösezeitpunktes für eine Alarmmeldung eine stetige Funktion verwendet, auf einfache Weise in bereits bestehende Gefahrmelder implementiert werden kann. Dazu ist es lediglich erforderlich, eine geeignete Software in die Auswerteeinrichtung, welche beispielsweise ein üblicher Prozessor zur Datenverarbeitung ist, zu laden.

Der beschriebene Gefahrmelder kann auch noch eine weitere Detektionseinrichtung aufweisen, welche zum Erfassen eines anderen, auf einer anderen physikalischen Messung beruhenden Messwertes dient. Wie oben im Zusammenhang mit einem verfahrensbezogenen Ausführungsbeispiel bereits erläutert, kann die weitere Detektionseinrichtung beispielsweise zum Messen einer Rauchkonzentration, einer Temperatur oder einer beliebigen anderen Messgröße verwendet werden, welche ebenfalls für ein Gefahrenpotential indikativ ist.

Der beschriebene Gefahrmelder kann ferner eine Sende- und Empfangseinheit aufweisen, welche zum drahtgebundenen und/oder zum drahtlosen Kommunizieren mit einer Zentrale eines Gefahrmeldesystems geeignet ist.

Der Auswerteeinrichtung kann auch ein nicht-flüchtiger Speicher des Gefahrenmelders zugeordnet sein. Im Falle der Bestimmung der stetigen Funktion durch einen oder durch mehrere Parameter können diese Parameter in dem nicht-flüchtigen Speicher gespeichert sein. Falls der Gefahrenmelder später andere Normen erfüllen soll, so ist es dann lediglich erforderlich, einen weiteren Satz von Parameterwerten in dem nicht-flüchtigen Speicher abzulegen.

Gemäß einem weiteren Aspekt der Erfindung wird ein Programm-Element zum Bestimmen eines Auslösezeitpunkts für eine Alarmmeldung eines Gefahrenmelders beschrieben. Das Programm-Element ist, wenn es von einer Auswerteeinheit ausgeführt wird, zum Durchführen des oben beschriebenen Verfahrens geeignet.

Das Programm-Element kann als computerlesbarer Anweisungscode in jeder geeigneten Programmiersprache wie beispielsweise in JAVA, C/C++ etc. implementiert sein. Das Programm-Element kann auf einem computerlesbaren Speichermedium (CD-Rom, DVD, Wechsellaufwerk, flüchtiger oder nicht-flüchtiger Speicher, eingebauter Speicher/ Prozessor etc.) abgespeichert sein. Der Anweisungscode kann einen Computer oder andere programmierbare Geräte derart programmieren, dass die gewünschten Funktionen ausgeführt werden. Ferner kann das Programm-Element in einem Netzwerk wie beispielsweise dem Internet bereitgestellt werden, von dem es bei Bedarf von einem Nutzer herunter geladen werden kann.

Im Sinne der vorliegenden Anmeldung ist die Erwähnung eines solchen Programm-Elements gleichbedeutend mit der Erwähnung eines Computerprogrammprodukts und/oder eines computerlesbaren Mediums, das Anweisungen zum Steuern eines Computersystems enthält, um die Arbeitsweise eines Gefahrenmelders in geeigneter Weise zu koordinieren, so dass die mit dem erfindungsgemäßen Verfahren verknüpften Wirkungen erreicht werden können.

Es wird darauf hingewiesen, dass die Erfindung sowohl mittels eines Computerprogramms, d.h. einer Software, als auch mittels einer oder mehrerer spezieller elektrischer Schaltungen, d.h. in Hardware oder in beliebig hybrider Form, d.h. mittels Software-Komponenten und Hardware-Komponenten, realisiert werden kann. Eine Implementierung der Erfindung mittels Software ist wegen ihrer Einfachheit bevorzugt.

Es wird ferner darauf hingewiesen, dass Ausführungsformen der Erfindung mit Bezug auf unterschiedliche Erfindungsgegenstände beschrieben wurden. Insbesondere sind einige Ausführungsformen der Erfindung mit Verfahrensansprüchen und andere Ausführungsformen der Erfindung mit Vorrichtungsansprüchen beschrieben. Dem Fachmann wird jedoch bei der Lektüre dieser Anmeldung sofort klar werden, dass, sofern nicht explizit anders angegeben, zusätzlich zu einer Kombination von Merkmalen, die zu einem Typ von Erfindungsgegenstand gehören, auch eine beliebige Kombination von Merkmalen möglich ist, die zu unterschiedlichen Typen von Erfindungsgegenständen gehören.

Weitere Vorteile und Merkmale der vorliegenden Erfindung ergeben sich aus der folgenden beispielhaften Beschreibung derzeit bevorzugter Ausführungsformen. Die einzelnen Figuren der Zeichnung dieser Anmeldung sind lediglich als schematisch und als nicht maßstabsgetreu anzusehen.

Figur 1 zeigt ein Gefahrmeldesystem, welches eine Zentrale und einen Gefahrenmelder mit einer Auswerteeinrichtung aufweist, die zum Ausführen des Verfahrens zum Bestimmen eines Auslösezeitpunkts für eine Alarmmeldung des Gefahrenmelders eingerichtet ist.

Figur 2 zeigt ein Diagramm, in dem zwei stetige Funktionen zusammen mit von einer Norm geforderten Alarmierungsbedingungen für einen CO-Gasmelder dargestellt sind.

Figur 3 zeigt ein Diagramm, in dem eine segmentierte stetige Funktion dargestellt ist, welche vier Knickstellen aufweist und welche die von einer Norm geforderten Alarmierungsbedingungen für einen CO-Gasmelder approximiert.

Figur 4 zeigt ein Diagramm, in dem durch die Norm EN 50291 definierte Alarmierungsbedingungen für einen CO-Gasmelder dargestellt sind.

An dieser Stelle wird darauf hingewiesen, dass sich die Bezugszeichen von in unterschiedlichen Figuren der Zeichnung dargestellten gleichen oder einander entsprechenden Komponenten lediglich in ihrer ersten Ziffer unterscheiden. Ferner wird darauf hingewiesen, dass die nachfolgend beschriebenen Ausführungsformen lediglich eine beschränkte Auswahl an möglichen Ausführungsvarianten der Erfindung darstellen. Insbesondere ist es möglich, die Merkmale einzelner Ausführungsformen in geeigneter Weise miteinander zu kombinieren, so dass für den Fachmann mit den hier explizit dargestellten Ausführungsvarianten eine Vielzahl von verschiedenen Ausführungsformen als offensichtlich offenbart anzusehen sind.

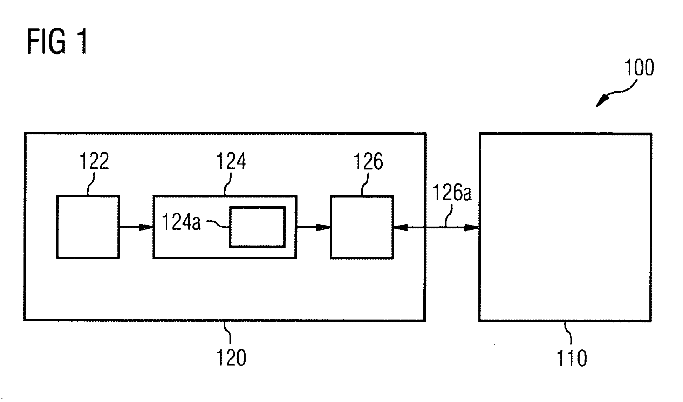

Figur 1 zeigt ein Gefahrmeldesystem 100, welches eine Gefahrmeldezentrale 110 und einen Gefahrenmelder 120 aufweist. Gemäß dem hier dargestellten Ausführungsbeispiel ist der Gefahrenmelder ein Gasmelder 120, welcher sensitiv ist für CO-Konzentrationen. Dies schließt jedoch keineswegs aus, dass der Gefahrenmelder 120 nicht auch noch für andere Gase und/oder für die Erfassung von anderen Gefährdungspotentialen wie beispielsweise Rauch sensitiv sein kann.

Zur Erfassung der Gaskonzentration weist der Gefahrenmelder 120 eine Detektionseinrichtung 122 auf. Die Detektionseinrichtung 122 weist einen nicht näher dargestellten für die Messung von CO-Konzentrationen geeigneten Gassensor auf.

Der Detektionseinrichtung 122 ist eine Auswerteeinrichtung 124 nachgeschaltet. Die Auswerteeinrichtung 124 weist eine Recheneinheit 124a auf, welche dazu eingerichtet ist, das in dieser Anmeldung beschriebene Verfahren zum Bestimmen eines Auslösezeitpunkts für eine Alarmmeldung des Gefahrenmelders 120 auszuführen. Der Gefahrenmelder 120 weist ferner eine Sende- und Empfangseinheit 126 auf, welche das von der Auswerteeinrichtung 124 bereit gestellte Resultat über eine Funk-Kommunikationsverbindung oder drahtgebundene Verbindung 126 an die Gefahrmeldezentrale 110 weiter leitet.

In der Recheneinheit 124a wird unter Verwendung einer stetigen Funktion f(A,B,C,D,E;t) bestimmt, ob und wann ein Alarm ausgegeben werden muss. Dabei sind A, B, C, D und E Parameter, durch welche die stetige Funktion so festgelegt ist, dass die die in dem jeweiligen Land gültige Norm in Bezug auf Auslöse- bzw. Wartezeiten erfüllt wird.

Im Folgenden werden einige Beispiele für die stetige Funktion genannt. Selbstverständlich müssen die Parameter A, B, C, D und E, sofern sie für die betreffende Funktion überhaupt verwendet werden, im Rahmen einer geeigneten Anpassungsprozedur an die jeweils gültige Norm angepasst werden.

$$f1(A,B,C,D;t) = [(A+t)/(B/t+C \cdot t)]+D$$

$$f2(A,B,C;t) = A \cdot arctan(B \cdot t)+C$$

$$f3(A,B,C,D;t) = A/[1+B \cdot exp(C \cdot t)]+D$$

$$f4(A,B,C;t) = 1/(A \cdot t+B)+C$$

$$f5(A,B,C,D;t) = A/[(1+B \cdot t^C)^2]+D$$

$$f6(A,B,C,D,E;t) = (A+B \cdot t)/(1+C \cdot t+D \cdot t^2)]+E$$

$$f7(A,B,C,D;t) = A \cdot exp\{[(ln(t)-B)^2]/C\}+D$$

$$f8(A,B,C,D;t) = A \cdot t^{[B+C \cdot ln(t)]}+D$$

$$f9(A,B,C,D,E;t) = A \cdot exp(B \cdot t)+C \cdot exp(D \cdot t)+E$$

In dieser Auflistung steht "/" für den Divisionsoperator, "^" für eine Potenzierung, "exp" für die Exponentialfunktion mit der Basis e und "ln" für den Logarithmus zur Basis e.

Bei einer von dem Erfinder durchgeführten Simulation hat sich ergeben, dass alle derzeit bekannten Normen für den Betrieb eines CO-Gasmelders durch ein und dieselbe stetige Funktion in guter Näherung approximiert werden können. Dies gilt für alle oben aufgeführten Funktionen f1 bis f9. Die Koeffizienten A, B, C und ggf. D und E sind dabei jeweils für unterschiedliche Normen verschieden, der Aufbau der jeweiligen Funktion f1 bis f9 kann aber für eine gute Approximation der durch verschiedene Normen vorgegebenen Alarmierungsbereiche unverändert bleiben. Somit kann der jeweilige Algorithmus zur Bestimmung der Wartezeit bzw. des Alarmauslösezeitpunkts für alle Normen, die der Gasmelder bzw. der Gas-Rauchmelder erfüllen muss, identisch bleiben und es muss lediglich die verwendete stetige Funktion f1, f2, ..., oder f9 ausgewertet werden, um abhängig von der CO-Konzentration den Zeitpunkt für eine Alarmmeldung zu bestimmen.

Es wird an dieser Stelle ausdrücklich darauf hingewiesen, dass die hier angegebenen Funktionen f1 bis f9 keine abschließende Aufzählung von möglichen Funktionen darstellen, welche für das in dieser Anmeldung beschriebene Verfahren zum Bestimmen eines Auslösezeitpunktes für eine Alarmmeldung geeignet sind.

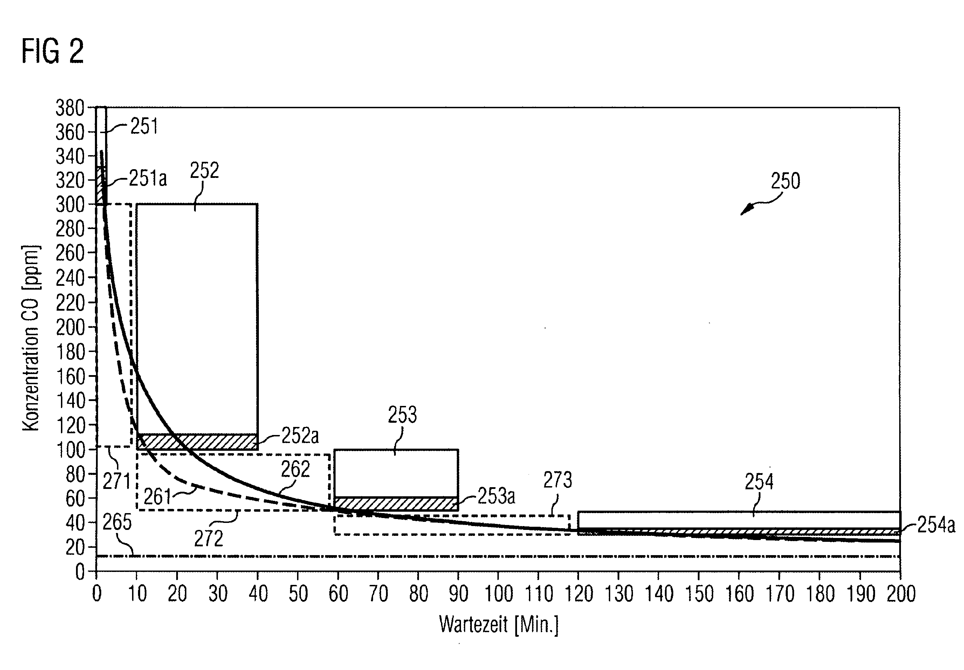

Figur 2 zeigt ein Diagramm 250, in dem die beiden oben aufgelisteten stetigen Funktionen f4 und f9 in einem Koordinatensystem aufgetragen sind, bei dem auf der Ordinate der Messwert bzw. die CO-Konzentration in der Einheit ppm und auf der Abszisse die Wartezeit aufgetragen ist, welche zusammen mit der jeweiligen Messzeit der Auslösezeitpunkt für eine Alarmmeldung bestimmt. Die Funktion f9 ist mit einer gestrichelten Linie dargestellt und mit dem Bezugszeichen 261 gekennzeichnet. Die Funktion f4 ist mit einer durchgezogenen Linie dargestellt und mit dem Bezugszeichen 262 gekennzeichnet.

Wie aus Figur 2 ersichtlich, sind die Parameter A, B und C der Funktion f4 und die Parameter A, B, C, D und E der Funktion f9 so gewählt, dass die von der Norm EN 50291 vorgegebenen Alarmierungsbedingungen, die erste Alarmierungsbedingung 251a, die zweite Alarmierungsbedingung 252a, die dritte Alarmierungsbedingung 253a und die vierte Alarmierungsbedingung 254a und die beispielhaft dargestellten Alarmierungsbereiche, der erste Alarmierungsbereich 251, der zweite Alarmierungsbereich 252, der dritte Alarmierungsbereich 253 und der vierte Alarmierungsbereich 254 sehr gut approximiert werden.

Die Parameter der Funktionen f4 und f9 sind dabei derart gewählt, dass für alle Alarmierungsbedingungen 251a, 252a, 253a und 254a jeweils die unterste horizontale Begrenzungslinie geschnitten wird. Die Stellen, an denen die betreffende Funktion f4 oder f9 diese Begrenzungslinien schneiden, definieren die jeweiligen maximalen Wartezeiten, die sich bei einem Überschreiten des jeweiligen Grenzwertes ergeben.

Zudem werden durch die Verwendung der stetigen Funktion f4 oder f9 ein erster ergänzter Alarmierungsbereich 271, ein zweiter ergänzter Alarmierungsbereich 272 und ein dritter ergänzter Alarmierungsbereich 273 abgedeckt. Diese ergänzten Alarmierungsbereiche ergeben eine schnellere Alarmierung als wenn nur die von der Norm vorgegebenen Alarmierungsbedingungen für die Alarmierung benützt würden. Durch die Verwendung einer stetigen Funktion f4 oder f9 wird in eindeutiger Weise jedem Messwert eine Wartezeit zugeordnet, wobei im Falle eines zeitlich ansteigenden Messwertes Unstetigkeiten bei der Bestimmung der tatsächlichen Alarmauslösezeit, die sich durch ein Hinzufügen der jeweiligen Wartezeit zu der jeweiligen Messzeit ergibt, vermieden werden.

In dem Diagramm 250 ist außerdem ein minimaler Grenzwert 265 für den Messwert bzw. für die CO-Konzentration angegeben. Dieser minimale Grenzwert 265 ist unabhängig von der Wartezeit. Der wartezeitunabhängige minimale Grenzwert 265 stellt somit eine absolute untere Schranke für die CO-Konzentration dar, unterhalb der eine Alarmauslösung nicht erfolgen darf. Der minimale Grenzwert 265 kann ebenfalls von gesetzlichen Normen vorgeschrieben sein, um unabhängig von einem zeitlichen Verlauf einer CO-Konzentration, welche jedoch stets kleiner ist als der minimale Grenzwert 265, ungewollte Falschalarme zu vermeiden.

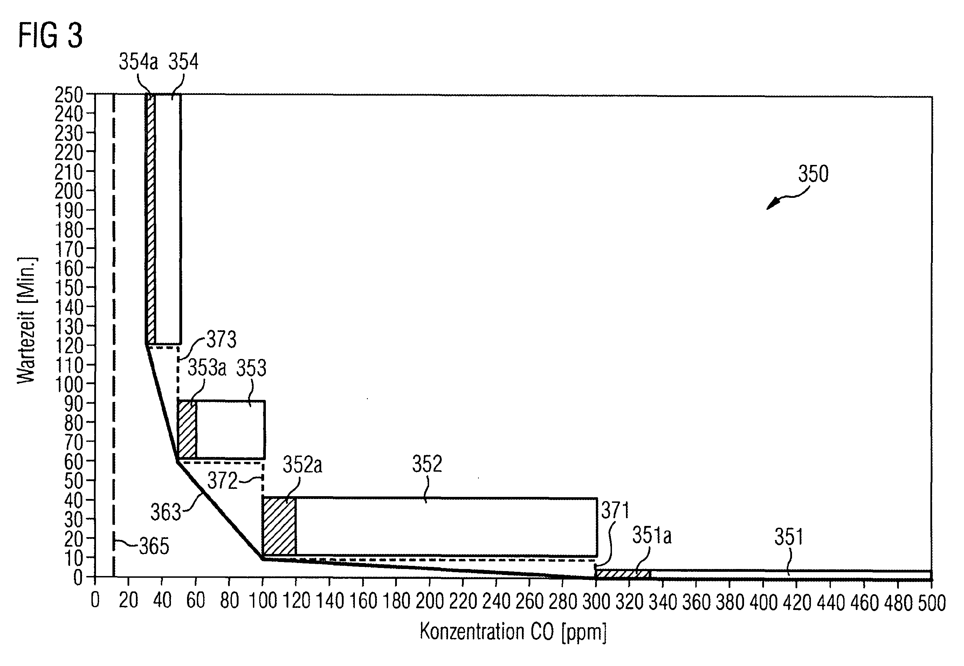

Figur 3 zeigt ein Diagramm 350, in dem eine segmentierte stetige Funktion g dargestellt ist, welche mit dem Bezugszeichen 363 versehen ist. Die Funktion 363 ist in einem Koordinatensystem aufgetragen, bei dem auf der Abszisse der Messwert bzw. die CO-Konzentration in der Einheit ppm und auf der Ordinate die Wartezeit aufgetragen ist. Die Wartezeit bestimmt zusammen mit der jeweiligen Messzeit den genauen Auslösezeitpunkt für eine Alarmmeldung.

Die Funktion 363 approximiert die von einer Norm geforderten Alarmierungsbedingungen für einen CO-Gasmelder. Die stetige Funktion 363 ist aus insgesamt drei linearen Teilfunktionen gi(ai,bi;Messwert), i = 1 bis 3, zusammengesetzt und weist somit Knickpunkte auf. Die linearen Teilfunktionen gi werden dabei durch die jeweils unteren linken Eckpunkte der normierten Alarmierungsbedingungen, einer ersten Alarmierungsbedingung 351a, einer zweiten Alarmierungsbedingung 352a, einer dritten Alarmierungsbedingung 353a und einer vierten Alarmierungsbedingung 354a, bestimmt. Die Alarmierungsbedingungen 351a, 352a, 353a und 353a sind dieselben wie die in der Figur 2 dargestellten und mit dem Bezugszeichen 251a, 252a, 253a und 254a Alarmierungsbedingungen. Das gleiche gilt für die den jeweiligen Alarmierungsbedingungen 351a, 352a, 353a und 354a respektiv zugeordneten Alarmierungsbereichen 351, 351, 351 und 351, welche von der Norm EN 50291 nicht vorgeschrieben sind.

Gemäß dem hier dargestellten Ausführungsbeispiel nimmt die Funktion g(a,b;Messwert) für unterschiedliche Messwerte bzw. unterschiedliche CO Konzentrationen folgende Werte an:

g(Messwert < 30 ppm CO): Der Messwert liegt unterhalb des durch die vierte Alarmierungsbedingung 354a vorgegebenen Grenzwerts. Die Funktion g ist somit für diese Messwerte nicht definiert. Alternativ kann derart kleinen Messwerten auch eine Wartezeit "unendlich" zugeordnet werden.

g(Messwert > 300 ppm CO)= 0: Bei einer derart hohen Konzentration erfolgt sofort eine Alarmierung.

g(Konzentration CO_i <= Messwert <= Konzentration CO_i+1)= = ai · Messwert + bi für i = 1 .. 3: Wie aus Figur 3 ersichtlich ergibt sich gemäß dem hier dargestellten Ausführungsbeispiel für CO_1 ein Wert von 30 ppm, für CO_2 ein Wert von 50 ppm, CO_3 ein Wert von 100 ppm und für CO_4 ein Wert von 300 ppm. Die Parameter ai und bi ergeben sich jeweils durch eine einfache Anpassung der jeweiligen Teilgeraden der Funktion g an die unteren linken Eckpunkte von zwei benachbarten Alarmierungsbedingungen.

Selbstverständlich kann eine geknickte stetige Funktion g auch durch mehrere Teilfunktionen gi zusammengesetzt werden. Dabei ist es auch möglich, dass zumindest einige der Teilfunktionen im Vergleich zu einer einfachen Geraden eine etwas komplexere mathematische Form aufweisen.

Wie aus Figur 3 ersichtlich, werden durch die Teilfunktionen gi ferner ergänzende Alarmierungsbereiche 371, 372 und 373 definiert, welche jeweils die Form eines Dreiecks aufweisen. Auch die ergänzten Alarmierungsbereiche 371, 372 und 373 ergeben eine schnellere Alarmierung als wenn nur die von der Norm vorgegebenen Alarmierungsbedingungen 351a, 352a, 353a und 354a für die Alarmierung benützt werden würden.

In dem Diagramm 350 ist ferner ein minimaler Grenzwert 365 für die CO-Konzentration angegeben, unterhalb der eine Alarmauslösung nicht erfolgen darf. Der minimale Grenzwert 365, welcher ebenfalls von gesetzlichen Normen vorgeschrieben sein kann, spielt in dem hier beschriebenen Ausführungsbeispiel jedoch keine Rolle, da die Funktion g lediglich in einem Messwertbereich größer oder gleich 30 ppm und damit deutlich oberhalb des dargestellten minimalen Grenzwertes definiert ist. Wie oben bereits beschrieben, erfolgt damit bei Messwerten kleiner als 30 ppm CO keine Alarmierung.

**Patentansprüche**

**1.** Verfahren zum Bestimmen eines Auslösezeitpunkts für eine Alarmmeldung eines Gefahrenmelders (120), das Verfahren aufweisend

● Erfassen eines Messwertes zu einem Messzeitpunkt, wobei der Messwert für ein Gefahrenpotential innerhalb eines Überwachungsbereiches indikativ ist,

● Ermitteln einer Wartezeit mittels einer stetig verlaufenden Funktion (261, 262) derart, dass die Wartezeit mit steigendem Messwert abnimmt und mit fallendem Messwert zunimmt, wobei bei einer geringfügigen Änderung des Messwertes sich auch nur geringfügig die Wartezeit ändert, und
● Bestimmen des Auslösezeitpunkts, indem die ermittelte Wartezeit zu der Messzeit hinzugefügt wird.

2. Verfahren nach dem vorangehenden Anspruch, wobei die Funktion (261, 262) derart beschaffen ist, dass sie innerhalb eines durch eine Norm vorgegebenen Wartezeitbereiches einen ebenfalls durch die Norm vorgegebenen zugehörigen unteren Grenzwert für den Messwert überschreitet.

3. Verfahren nach einem der vorangehenden Ansprüche, wobei die Funktion (261, 262) derart beschaffen ist, dass sie einen wartezeitunabhängigen minimalen Grenzwert (265) für den Messwert nicht unterschreitet.

4. Verfahren nach einem der vorangehenden Ansprüche, wobei die Funktion (261, 262) derart beschaffen ist, dass sie sich innerhalb eines Messwertbereiches mit besonders hohen Messwerten asymptotisch an die Wartezeit Null annähert.

5. Verfahren nach einem der vorangehenden Ansprüche, wobei die Funktion (261, 262) durch einen oder durch mehrere Parameter und durch eine Funktionsvariable bestimmt wird.

6. Verfahren nach dem vorangehenden Anspruch, wobei der oder die Parameter derart gewählt sind, dass die Funktion (261, 262) einen von der Wartezeit unabhängigen konstanten Messwert ergibt, welcher einen Grenzmesswert darstellt

7. Verfahren nach einem der beiden vorangehenden Ansprüche, wobei
die Funktionsvariable

- die Wartezeit oder
- der Messwert ist.

8. Verfahren nach einem der vorangehenden Ansprüche, wobei die Funktion (363) mehrere Teilabschnitte aufweist, wobei jeweils zwei benachbarte Teilabschnitte an einer Knickstelle der Funktion (363) miteinander verbunden sind.

9. Verfahren nach einem der vorangehenden Ansprüche, wobei der Messwert indikativ ist für die Konzentration eines Gases.

10. Gefahrenmelder zum Auslösen einer Alarmmeldung, der Gefahrenmelder (120) aufweisend

● eine Detektionseinrichtung (122), eingerichtet zum Erfassen eines Messwertes, welcher für ein Gefahrenpotential innerhalb eines Überwachungsbereiches indikativ ist, und
● eine Auswerteeinrichtung (124), welche mit der Detektionseinrichtung (122) gekoppelt ist und welche derart eingerichtet ist, dass das Verfahren nach einem der vorangehenden Ansprüche ausführbar ist.

11. Programm-Element zum Bestimmen eines Auslösezeitpunkts für eine Alarmmeldung eines Gefahrenmelders (120), das, wenn es von einer Auswerteeinheit (124) ausgeführt wird, zum Durchführen des Verfahrens nach einem der Ansprüche 1 bis 9 geeignet ist.


## Claims

1. Method for determining a triggering time for an alarm of an alarm device (120), with said method featuring

● detection of a measured value at a measuring time, with the measured value being indicative of a hazard potential within a monitoring range,
● establishing a waiting time by means of a permanently running function (261, 262) such that the waiting time reduces as the measured value rises, and increases as the measured value falls, wherein the waiting time also only changes slightly if a slight change is made to the measured value, and
● determination of the triggering time by the waiting time established being added to the measuring time.

**2.** Method in accordance with the preceding claim, with the function (261, 262) being constituted such that, within a waiting time range predetermined by a standard, it exceeds an associated lower threshold for the measured value likewise predetermined by the standard.

**3.** Method in accordance with one of the preceding claims, with the function (261, 262) being constituted such that it does not fall below a waiting-time-independent minimum threshold (265) for the measured value.

**4.** Method in accordance with one of the preceding claims, with the function (261, 262) being constituted such that, within a measured value range with especially high measured values, it asymptotically approaches the waiting time of zero.

**5.** Method in accordance with one of the preceding claims, with the function (261, 262) being determined by one or by a number of parameters and by a function variable.

**6.** Method in accordance with the preceding claim, with the parameter or the parameters being selected such that the function (261, 262) produces a constant measurement value independent of the waiting time, which represents a measured value threshold.

**7.** Method in accordance with one of the two preceding claims, with the function variable

- being the waiting time or
- the measured value.

**8.** Method in accordance with one of the preceding claims, with the function (363) featuring a number of subsections, with two adjacent subsections being connected to one another at an inflection point of the function (363).

**9.** Method in accordance with one of the preceding claims, with the measured value being indicative for the concentration of a gas.

**10.** Alarm device for issuing an alarm, with the alarm device (120) featuring

● a detection device (122), configured for detecting a measured value which is indicative for a hazard potential within a monitoring range, and
● an evaluation device (124), which is coupled to the detection device (122) and which is configured such that method in accordance with one of the preceding claims is able to be executed.

**11.** Program element for specifying a triggering point for an alarm by an alarm device (120) which, when it is executed by an evaluation unit (124), is suitable for carrying out the method as claimed in one of claims 1 to 9.

**Revendications**

**1.** Procédé pour déterminer un moment de déclenchement pour un message d'alarme d'un avertisseur de danger (120), lequel procédé présente les étapes suivantes :

● enregistrement d'une valeur de mesure à un instant de mesure, la valeur de mesure étant indicative pour un potentiel de danger à l'intérieur d'une zone de contrôle,
● détermination d'un délai d'attente au moyen d'une fonction (261, 262) fonctionnant de façon continue de telle sorte que le délai d'attente diminue avec l'accroissement de la valeur de mesure et augmente avec la diminution de la valeur de mesure, sachant que, dans le cas d'une variation minime de la valeur de mesure, le temps d'attente ne varie également que légèrement, et
● détermination du moment de déclenchement du fait que le délai d'attente déterminé est ajouté au temps de mesure.

**2.** Procédé selon la revendication précédente, la fonction (261, 262) est configurée de façon qu'elle dépasse, dans les limites d'une plage de temps d'attente prédéfinie par une norme, une valeur limite inférieure spécifique, prédéfinie également par la norme, pour la valeur de mesure.

**3.** Procédé selon l'une des revendications précédentes, la fonction (261, 262) étant configurée, de telle sorte qu'elle ne descend pas au-dessous d'une valeur limite (265) minimale indépendante du temps d'attente pour la valeur de mesure.

**4.** Procédé selon l'une des revendications précédentes, la fonction (261, 262) étant configurée de telle sorte qu'elle se rapproche, dans les limites d'une plage de valeurs de mesure, avec des valeurs de mesure particulièrement élevées, de façon asymptotique du délai d'attente zéro.

**5.** Procédé selon l'une des revendications précédentes, la fonction (261, 262) étant déterminée par un ou plusieurs paramètres et par une variable de fonction.

**6.** Procédé selon la revendication précédente, le ou les paramètres étant choisi(s) de telle sorte que la fonction (261, 262) donne une valeur de mesure constante, indépendante du délai d'attente, qui représente une valeur de mesure limite.

**7.** Procédé selon l'une des deux revendications précédentes,
la variable de fonction étant

- le délai d'attente ou
- la valeur mesurée.

**8.** Procédé selon l'une des revendications précédentes, la fonction (363) présentant plusieurs tronçons partiels, à chaque fois deux tronçons partiels aux voisins étant reliés l'un à l'autre en un point de flexion de la fonction (363).

**9.** Procédé selon l'une des revendications précédentes, la valeur de mesure étant indicative pour la concentration d'un gaz.

**10.** Avertisseur de danger pour le déclenchement d'un message d'alarme, l'avertisseur d'alarme (120) présentant

● un dispositif de détection (122) équipé pour l'enregistrement d'une valeur de mesure, laquelle est indicative pour un potentiel de danger à l'intérieur d'une zone de contrôle, et
● un dispositif d'analyse (124), qui est couplé avec le dispositif de détection (122) et qui est équipé de telle sorte, que le procédé selon l'une des revendications précédentes peut être mis en oeuvre.

**11.** Elément de programme pour la détermination d'un moment de déclenchement pour un message d'alarme d'un avertisseur de danger (120), lequel, lorsqu'il est mis en oeuvre par une unité d'analyse (124), convient pour la mise en oeuvre du procédé selon l'une des revendications 1 à 9.

## FIG 1

# FIG 2

FIG 3

EP 2 297 717 B1

# FIG 4
## Stand der Technik

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4088986 A
- WO 2005119618 A2
- EP 0880764 B1